# EUROPÄISCHE PATENTSCHRIFT

(11) **EP 1 753 375 B1**
(45) Veröffentlichungstag und Bekanntmachung des Hinweises auf die Patenterteilung: **10.10.2012**
(21) Anmeldenummer: 05756998.0
(22) Anmeldetag: 07.06.2005
(51) Int. Cl.: A61F 2/28, A61L 27/56

(54) **GEKAMMERTER WERKSTOFF ALS IMPLANTAT, KNOCHENERSATZ UND ALLGEMEIN ALS WERKSTOFF**
POROUS MATERIAL FOR USE AS IMPLANT, BONE REPLACEMENT AND IN GENERAL AS MATERIAL
MATIERE PREMIERE POREUSE SERVANT D'IMPLANT, DE SUBSTITUT OSSEUX ET DE MANIERE GENERALE DE MATIERE PREMIERE

(30) Priorität: 07.06.2004 DE 102004027657
(43) Veröffentlichungstag der Anmeldung: 21.02.2007
(60) Teilanmeldung: 10169578.1
(73) Patentinhaber: BoneArtis AG, 6440 Brunnen (Sz) (CH)
(72) Erfinder: DRAENERT, Klaus, 81545 München (DE)
(74) Vertreter: Vossius & Partner
(86) Internationale Anmeldenummer: PCT/EP2005/006115
(87) Internationale Veröffentlichungsnummer: WO 2005/120399

(56) Entgegenhaltungen:
- WO-A-95/21053
- US-A- 3 899 556
- US-B1- 6 316 091

## Beschreibung

In der Chirurgie am Bewegungsapparat besteht seit jeher ein Bedarf, Knochendefekte nach Frakturen, nach Entfernung von Tumoren, Knochensubstanzverluste nach Entzündungen oder in Verbindung mit Knochenzysten mit Knochenmaterial oder mit einem dem Knochen ähnlichen Ersatzmaterial wieder aufzufüllen. Hierbei wurde zum Teil auch auf bovines Ersatzmaterial zurückgegriffen [Tröster SD 1993: Die Hydroxylapatitkeramik Endobon®). Eine alternative Therapiemöglichkeit für Knochendefekte. In: Venbrocks R, Salis G von (Hrsg): Jahrbuch der Orthopädie,pp 231-246. Zülpich: Biennann ] oder Material aus Korallenriffen [Irwin RB, Bernhard M, Biddinger A (2001) Coralline hydroxyapatite as bone substitute in orthopaedic oncology. Am J Orthop 30:544-550] im Labor aufbereitet (replaminiforme Prozesse) und eingesetzt. Beiden Materialien haftet der Makel an, dass es biologische und damit gerichtete Strukturen sind, die man nicht mehr beeinflussen kann und die kaum standardisiert herstellbar sind. Beide Werkstoffe bringen die Anisotropie in Struktur und Eigenschaften einer gewachsenen biologischen Struktur mit und sind in fast allen Fällen zu steif. Ein Verfahren für die Herstellung solch biologischen Ersatzmaterials ist in der DE-A 3903695 beschrieben. Synthetische Ersatzwerkstoffe sind zwar aus reinen Ausgangsmaterialien herstellbar, lassen jedoch eine geordnete, dem Knochen ähnliche Struktur vermissen; ein solcher synthetischer Werkstoff ist das geschäumte Ceros®; diese Werkstoffe lassen einen knöchernen Durchwuchs vermissen, da die Poren meist geschlossen sind [Dingeldein E und H Wahlig (1992) Fluoreszenzmikroskopische Untersuchungen zur knöchernen Integration von Kalziumphosphatkeramiken In: Merck Biomaterialien (Hrsg) Endobon® und DBCS®, Darmstadt:Merck ].

In DE-A-2242 867 und US-A- 38 99 556 wird ein Verfahren mit einem präformierten, formgebenden Gerüst beschrieben mit einer dichten Schüttung von Kugeln, bei dem die formgebenden Elemente von einem Lösungsmittel übergossen werden und auf diese Weise verklebt werden sollen; mit einem solchen Verfahren lässt sich jedoch kein gieichmässig poröses interkonnektierendes Material in stardardisierter Form herstellen; zu unregelmässig waren die Verklebungen und zu unkontrollierbar der Anlösungsprozess.
In EP-A-0 553167 und EP-A-0204786 wurde das Problem teilweise gelöst, indem deformierbare formgebende Elemente durch Druckbeaufschlagung in Kontakt aufeinander gepresst wurden und in diesem Zustand von der Gerüst-bildenden Masse umgossen wurden, welche danach aushärtete und anschliessend von den formgebenden Elementen chemisch oder thermisch wieder befreit wurde. Die so hergestellten Implantate liesen in der Hälfte, die der Druckbeaufschlagung zugewandt war, schöne und nahezu gleichmässige Interkonnektierungen erkennen, in der von dieser abgewandten Hälfte jedoch waren zahlreiche geschlossenen Poren ausgebildet. Formgebende deformierbare und nur teilweise elastischen Elemente dämpfen die einwirkende Kraft und die deformierende Wirkung erschöpft sich durch Dämpfung, sodass keine gleichmässige und durchgehende Interkonnexion erreicht wird. Rein zufällig wurde nun ein Verfahren gefunden, welches diese Phänomene vermeiden lässt und vollständig gleichmässige Verformung aller am Prozess beteiligten formgebenden Elemente erreichen lässt. Dieses Verfahren ist denkbar einfach und daher auch besonders preiswert und vollständig reproduzierbar.
Aus WO 95/21053 A ist ein Verfahren zur Herstellung eines Werkstoffs mit einem Gerüst aus schalenartigen Strukturen und einem interkonnektierenden Porensystem bekannt. Bei diesem Verfahren werden deformierbare Formkörper in eine Form zur Ausbildung einer Schüttung der deformierbaren Formkörper eingefüllt, die Form wird mit einem gieß-, spritz- oder schüttfähigen Material befüllt und das Material verfestigt, und die deformierbaren Formkörper werden entfernt, wobei das Material das Gerüst aus schalenartigen Strukturen mit einem interkonnektierenden Porensystem bildet.

Aus US 6,316,091 B1 ist ein Verfahren zum Herstellen eines synthetischen Knochenersatzes bekannt, mit dem eine makroporöse synthetische Keramik mit Poren mit kontrollierten Abmessungen hergestellt werden soll, die bezüglich Anzahl und Oberfläche in einer vorbestimmten Weise verteilt sein sollen, wobei die Interkonnektion zwischen den Poren kontrolliert werden soll.

Die Erfindung ist durch die Merkmale des Implantatkörpers und seines Herstellungsverfahrens gemäß der Patentansprüche gekennzeichnet.

Die Anwendung von einem auf die formgebenden Elemente einwirkenden Unterdruck führte überraschend zu einem perfekten Ergebnis. Dabei werden die locker in ein Werkzeug geschütteten formgebenden Elemente entweder vor der Abfüllung in einem vakuumdichten System mit einem definierten Unterdruck beaufschlagt und anschliessend die gerustbildende Masse eingesaugt und unter Einwirkung des definierten Unterdruckes bei definierter Temperatur über eine definierte Zeit aushärten gelassen, oder nach der Abfüllung, zusammen mit dem Gerüstwerkstoff, mit definiertem Unterdruck beaufschlagt und gleichzeitig abgekühlt, beispielsweise über eine metallene Stellplatte:
Diese fast noch einfachere Anordnung führte zu weiter standardisierbaren Ergebnissen und beinhaltet folgende Schritte: die lockere Schüttung von formgebenden Elementen wurde mit der strukturbildenden Gerüstmasse umgossen und das geschlossene Gefäss, welches nicht vakuum - versiegelt war, wurde in einem dichten mit Unterdruck beaufschlagten Gefäss über eine bestimmte Zeit bei definierter Temperatur einem definierten Unterdruck ausgesetzt und gleichzeitig über die Stellplatte abgekühlt. Das einfache Handling dieses Vorgehens liess das Ergebnis hoch reproduzierbar werden.
Doch auch dieses Ergebnis konnte zufällig durch eine zunächst unscheinbare Veränderung, vor allem im Hinblick auf die einstellbare Porosität der Oberfläche, weiter verbessert werden: Nahm man nämlich statt eines festen metallischen Werkzeuges eine deformierbare Silikonform, so führte dies dazu, dass das Implantat, abhängig vom angelegten Unterdruck eine durchgehende bis in die Oberfläche reichende Porosität aufwies, die durch die Höhe des Unterdruckes auf der einen Seite und den E-Modul des Werkzeuges auf der anderen Seite einstellbar war. Dieses Verfahren liess auch dann eine durchgehende Porosität erreichen, wenn die formgebenden Elemente nicht expansionsfähig, also beispielsweise nicht lufthaltig, sondern beispielsweise Zuckerkugeln waren.

Als formgebende Elemente werden bei diesem Verfahren vorzugsweise expandierbare Polystyrol-Kugeln (EPS) verwendet, beispielsweise Styropor® F414, das mit Pentan als Treibmittel aufgeschäumt ist. Beim Anlegen eines Unterdruckes dehnen sich diese Kugeln sehr schnell aus und nehmen an Volumen zu. Auf diese Weise wird die Kontaktbrücke zwischen den Kugeln breiter und bestimmt damit die Durchmesser der interkonnektierenden Durchtritte bis hinein in die Oberfläche; bei Verwendung eines Silikonwerkzeuges drücken sich die Kugeln in die Silikonwand und zudem zieht der Unterdruck die deformierbare Wand über die Kugeloberfläche in das Implantat hinein.

Geschäumte Werkstoffe zur Verwendung als formgebende Elemente werden bevorzugt eingesetzt, auch solche, denen Treibmittel zugesetzt sind, welche bei einer bestimmten Temperatur oder unter bestimmten Voraussetzungen aktiviert werden. Ein besonders bevorzugtes Material stellt das Styropor® F414 dar mit bevorzugten Raumgewichten des aufgeschäumten Polystyrols zwischen 17g/l und 70g/l, vorzugsweise etwa 20g/l bis 35g/l. Die Korngrössenverteilung des aufgeschäumten Materials liegt zwischen 200µm und 15mm, häufig verwandt sind die Grössen zwischen 1000µm und 3000µm. Zur Bestimmung der Expansion und der Deformierbarkeit der einzelnen formgebenden Elemente wurden Versuche durchgeführt, um in einfacher Weise die Parameter zu bestimmen, die zur Standardisierung des Verfahrens notwendig sind. Hierfür wurden verschiedene formgebende Elemente mit verschiedenen Raumgewichten, beispielsweise verschieden geschäumte Polystyrol-Kugeln mit verschiedenen Durchmessern, in einem Zylinder mit beweglichen, vakuumdicht anliegenden Kolben bis zu einer definierten Höhe von 84,3mm aufgeschüttet und einem definierten Vakuum ausgesetzt ; aus der Veränderung der Ausgangshöhe in Abhängigkeit vom angelegten Unterdruck wurden Grössen ermittelt, die eine anfängliche Volumenreduktion durch Entfernen der Luft zwischen den formgebenden Elementen widerspiegeln, gefolgt von einer Volumenausdehnung, die durch Aufbringen einer Kraft F, gemessen in N, zur alten Ausgangslänge einstellbar war und den Expansionsdruck der Luft in den formgebenden Elementen widergab. In Abhängigkeit von der Zeit nahm die Kraft langsam ab, was durch das Platzen der Luftblasen im Kunststoff zu erklären war. Aus diesem Phänomen wurden die definierten Zeiten ermittelt für die Beaufschlagung mit Unterdruck.
Drücke zwischen 150mbar und 800mbar, vorzugsweise um 300mbar bis 500mbar über eine Zeit von 15 Minuten einwirkend auf ein Calciumphosphat - Agar-Agar Gemisch, bei einer Temperatur des Implantates von 4-12° C zeigten besonders günstige Ergebnisse in Bezug auf die Aussenporosität und die inneren Interkonnexionen.
Als deformierbare Formen eignen sich vor allem giess- oder im Spritzgussverfahren verwendete Silikone einer Shorehärte unter 25 Shore, vorzugsweise unter 18-20Shore. Es können aber alle plastisch oder elastisch deformierbaren Werkstoffe Verwendung finden, deren E-Modul deutlich unter dem der formgebenden Elemente liegt. Entsprechend können die Werkzeug gegossen werden, aber auch in grossen Serien im Spritzgussverfahren hergestellt werden. Beispiele für plastisch deformierbare Werkzeuge sind Werkzeuge aus Styropor® verschiedener Dichte, Beispiele für elastisch deformierbare Werkzeuge sind die oben erwähnten Silikone, wobei auch geschäumte Silikone zur Anwendung kommen können.
Der Expansionsdruck dieser Werkstoffe im Vakuum addiert sich in seiner Wirkung zu dem der formgebenden expansionsfähigen Elemente.

Als giessfähiges Material für den Gerüstwerkstoff wird vorzgsweise eine Mischung aus Hydroxylapatit (HA) oder Tricalciumphosphat (TCP) und einer Agar-Agar Lösung im Verhältnis 10g Pulver / 7ml bis zu 25ml Lösung, was einem Verhältnis von 1,4 bis 0,4 entspricht, ganz optimal sind Ansätze, bei denen das Mischungsverhältnis Pulver zu Lösung 0,45 bis 0,48 entspricht, beispielsweise 1600g HA auf 3500ml Lösung.

Je nach Zusammensetzung kann aus dem Ansatz bereits der Schrumpfungsfaktor berechnet werden: dieser liegt bei einem giessfähigen HA-Agar-Agar Gemisch, welches bei 60°C abgefüllt wird zwischen 0,95 bis 2,9, vorzugsweise zwischen 1,75 und 2,15, bei einem Verhältnis von 16g/35ml einer 1,7%igen Agar-Agar Lösung bei exakt 1,91. Mit diesen beiden Voraussetzungen, expandierbare formgebende Elemente, einer deformierbaren Silikonform und einem exakten Ansatz mit definierter Schrumpfung liessen sich sehr präzise Implantate "net shaped" sintern, ohne dass eine Nachbearbeitung notwendig wurde.

Das definitive Design multipliziert mit dem Schrumpfungsfaktor ergibt beispielsweise ein Implantatkörper in Kunststoff oder einem anderen im CAD/CAM Verfahren leicht bearbeitbaren Material, welches in einem Stammform bis zur Oberkante mit einem giessfähigen Silikon umgossen wird. Nach Aushärten des Silikons kann der formgebende Körper leicht wieder mechanisch entfern werden, die Silikonform wird am Boden mehrfach perforiert und danach mit Polystysrolkugeln der gewünschte Grösse aufgeschüttet, danach mit einem Silikondeckel mit Entlüftungslöchern verschlossen und in einer Abfüllform mit beispielsweise der Keramikmasse abgefüllt. Unmittelbar nach der Abfüllung wird das Werkzeug in toto in einem Exsiccator mit einem Unterdruck beaufschlagt und auf 4-12°C heruntergekühlt, beispielsweise über die Stellplatte. Nach Aushärten der Keramikmasse kann das Werkzeug demontiert werden und der Grünling ist leicht zu entfernen. Im Acetonbad wird nun aus dem Keramik-Styropor®-Implantat das Styropor herausgelöst, die Keramik dehydratisiert in Schritten 70/80/90 und 100%iges Aceton und anschliessend unter Kühlung schrittweise an Luft getrocknet (Kältetrocknung). Das Ergebnis wird stundenweise mit Hilfe einer Feinwaage dokumentiert, sowie kein weiterer Gewichtsverlust an Luft sich abzeichnet und die Gewichtskurve linear gleich bleibt, wird das Implantat über 24h im Exsiccator unter Zusatz von P₂O₅ unter Vakuum 150- 250 mbar Absolutdruck getrocknet und anschliessend bei 1300°C im Sinterofen gebrannt. Das Ergebnis ist ein offenporiges, absout masshaltiges Implantat mit einer gegenüber mechanisch nachbearbeiteten Knochenersatzwerkstoff-Zylindern oder Würfeln (Draenert et.al.2001:Synthetische Knochenersatzwerkstoffe auf HA- und TCP-Basis Trauma Berufskrh 3:293-300 Heidelberg New York Berlin Tokyo: Springer) deutlich höheren Festigkeit, die durch äussere Strukturierung weiter vergrössert werden kann, beispielsweis Ringe, Einschnürungen, massive Kanten usw.

### Beispiel 1

Styropor®-Kugel von 12mm Durchmesser werden in einem zylindrischen Gefäss mit perforiertem Boden mit Bohrungen von 10-11mm im Durchmesser und einem schlüssig den Kugeln aufsitzenden fixierbaren Deckel mit denselben Bohrungen in einen Becher getaucht und intrudiert, der mit 90°C heissem Wachs gefüllt und hierfür aus dem Heizofen genommen wird Der Behälter mit den Styropor®-Kugeln passt dabei mit seinem Aussendurchmesser schlüssig in das zylindrische Wachsgefäss, welches einen demontierbaren Boden aufweist. Nach Aushärten des Wachses bei Zimmertemperatur, wird der Boden des Wachsgefässes demontiert und der Styropor®-Behälter herausgedrückt. Der Styropor®-Behälter wird ebenfalls von seinem Boden und dem Deckel befreit und der Wachs-Styropor®-Zylinder herausgeschoben und in einem Acetonbad von dem Styropor® befreit. Nach Trocknung an Luft wird das so entstandene durchgehend interkonnektierend poröse Wachsgerüst in eine dieses schlüssig aufnehmendes Abfüllgefäss eingeschoben. Das Abfüllgefäss weißt einen Einfüllstutzen mit Perforationen am Boden des Gefässes auf und kann ausserdem ein Sieb aufnehmen, weiches zum Einsaugen kleinerer Styropor®-Kugeln benötigt wird, ausserdem ist es mit einem Deckel versehen, welcher Entlüftungsbohrungen aufweist, beispielsweise mit einem Durchmesser von 1,5 - 2,5mm. In das Wachsgerüst werden Styropor®-Kugeln mit einem Durchmesser von beispielsweise 600-1200µm eingesaugt, wobei im Abfüllstützen ein Sieb mit einer Maschenweit von 400µm vorgeschalten wird. Das Hohlraumsystem des Wachsgerüstes füllt sich vollständig mit den kleineren Styropor®-Kugeln auf und wird danach mit einem Deckel verschlossen. Das Gefüss wird nun mit einer Keramikmasse abgefüllt, wobei über den Abfüllstutzen die Masse über die Belüftungslöcher eingesaugt oder ohne grossen Druck einfach über den Stutzen aufgefüllt wird. Tritt die Keramikmasse aus den Entlüftungslöchern des Deckels aus, ist die Form gefüllt und wird nun mitsamt dem Werkzeug in einen Exsiccator gestellt und über 15 Minuten mit einem Unterdruck von 500-600mbar beaufschlagt und während dieser Zeit über die metallene Stellplatte abgekühlt. Der so ausgehärtete Wachs-Keramik-Styropor®-Block wird anschliessend im Acetonbad vom Styropor® befreit, an Luft getrocknet und mit dem Wachs im Ofen bei 1300°^{C} gesintert. Das Ergebnis sind vollständig isolierte, aussen durchgehend poröse und im Innern vollstandig interkonnektierend gekammerte Kugeln mit einem Durchmesser um 6mm. Aufgrund der Schrumpfung des Matrixmaterial kommt es zur Ausbildung vollständig separierter Kugeln.

### Beispiel 2

Statt einzelner gekammerter Kugeln kann ein interessanter Werkstoff gefertigt werden, der einem Negativabdruck der Knochenmarkswaben entspricht und ein Kugelkonglomerat mit Zwischenräumen zwischen den gekammerten Kugeln für das Einwachsen von Knochenbälkchen und breiten Verbindungsbrücken, die den Kontaktstellen der Kugeln entsprechen: Die Schritte entsprechen dem Beispiel 1, jedoch wird das Wachs-Styropor®-Gerüst während des Aushärtens in einem Exsiccator einem definierten Unterdruck von beispielsweise 600mbar ausgesetzt. Die daraufhin erfolgende Expansion der Sryropor®-Kugeln führt zu verbreiterten Brücken und dickeren. Verbindungsarmen zwischen den Kugeln. Nach Aushärten des Wachs-Styropor®-Gerüstes erfolgt das Herauslösen des Styropor®. Die nachfolgenden Schritte entsprechen dem Beispiel 1 mit Einsaugen von kleineren Styropor®-Kugeln, Auffüllen mit einer Keramikmasse, Nachevakuieren unter Abkühlen und anschliessendern Herauslösen der form gebenden Elemente im Aceton; danach erfolgt im Gegensatz zum Beispiel 1 der Kält-Trocknungsschritt: in Schritten von jeweils 2h wird über 70/80/90 und 3x 100 %iges Aceton in Stufen in der Kühltruhe an Luft getrocknet, bei 4-12°C aufsteigend in beispielsweise 4 Schritten von jeweils 3 Stunden bis zur Zimmertemperatur und anschliessender Dehydratation im Exsiccator unter Zuhilfenahme von P₂O₅ und einem Vakuum um 150mbar Absolutdruck über 24h. Bei einem solchen Vorgehen bleiben breite Brücken zwischen den gekammerten Kugeln bestehen und das Wachsgerüst kann mitsamt dem Keramikinlet im Ofen gebrannt werden bei beispielsweise 1300°C; das Wachs kann auch vorher im Heizofen abgeschmolzen werden bei 90°C, Voraussetzug hierfür ist, dass die Keramikmasse bereits luftgetrocknet ist. Das Ergebnis ist ein perfektes Gerüst aus zusammenhängenden im Inneren perfekt interkonnektierend gekammerten Kugeln mit durchgehend poröser Oberfläche und einer erstaunlich hohen Kompressionsfestigkeit. Diese liegt je nach Kugelgrösse bei 4-12 MPa.

### Beispiel 3

Zur Herstellung eines masshaltigen Würfels mit einer Kantenlänge von 15mm und einer offenen Porosität über alle Flächen. wird bei einer Matrixmasse von 16/35 HA/-Agar-Agar Suspension 1,7%ig ein Schrumpfungsfaktor von 1,91 berechnet und im CAD CAM Verfahren ein Würfel aus POM mit einer Kantenlänge von 28,65mm hergestellt. Da der Würfel eine hohe Festigkeit von 4-6 MPa erreichen soll, werden die Kanten nicht abgerundet. Der Würfel wird in ein Formwerkzeug gestellt und bis zu seiner Oberkante mit einem selbsthärtenden Silikon ausgegossen. Nach 24h wird der Würfel mechanisch entfernt und das Silikonwerkzeug in ein Abfüllwerkzeug eingebracht, dessen Boden innen aus einem perforierten Silikonboden besteht und nach Auffüllen mit 1200µm messenden Styropor®-Kugeln mit einem Silikondeckel mit Entlüftungslöchern schlüssig abgedeckt. Nachdem das Werkzeug mit einem Schraubdeckel mit Entlüftungslöchern verschlossen wurde, wird es mit einer Keramimasse aufgefüllt; anschlisend erfolgt im Exsiccator auf einer metallenen und kühlbaren Stellplatte die Beaufschlagung mit Unterdruck von 500mbar über 15 Minuten bei gleichzeitiger Abkühlung. Danach wird das Werkzeug demontiert und der Keramik-Styropor®-Würfel vorsichtig mechanisch herausgenommen und im Acetonbad von den formgebenden Elementen befreit. Zur rissfreien Trocknung wird die Kältedehydratisierung angeschlossen wie in den Beispielen 1 und 2 beschrieben und anschliessend der Würfel im Ofen bei beispielsweise 1300°C gesintert. Das Ergebnis ist ein masshaltiger sehr kompressionsstabiler Würfel mit offener Porosität über alle Flächen und einer durchgehend interkonnektierenden Porosität der inneren Struktur und verstärkten Kanten aus kompakter Keramik.
Die Erfindung wird nachstehend anhand der Figuren noch näher erläutert.

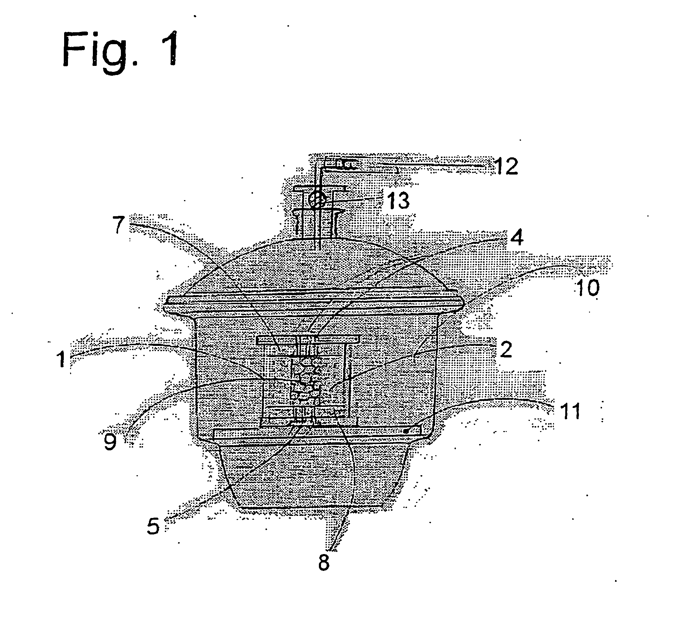

In Figur 1 ist eine einfache Vakuumkammer beschrieben in Form eines Exisccators 10 mit Belüftungsventil 13 und Hahn zum Anschluss eines Schlauches zur Vakuumpumpe 12 und einer metallenen Kühlplatte 11 mit einem auf der Kühlplatte stehenden Abfüllwerkzeug 1 mit einem schlüssig aufgenommenen deformierbaren Werkzeug in drei Teilen, einem Korpus 2, einem Deckel 7 mit Perforationen 4 und einem Boden 8 mit Perforationen 5 zum Auffüllen beispielsweise mit einer keramischen Masse.

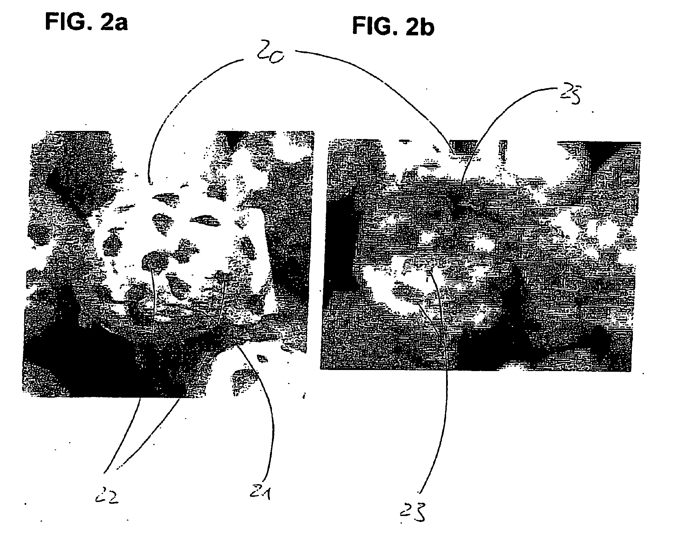

In Figuren 2a und 2b sind erfindungsgemäße keramische Implantate 20 dargestellt, gemäß Figur 2a in der Aufsicht und in Figur 2b als Bruch. Die Aufsicht auf die Kugelschale lässt die offene Porosität 22 erkennen und die raue Schale 21. Im Bruch ist die interkonnektierende Porenstruktur 23 dargestellt.

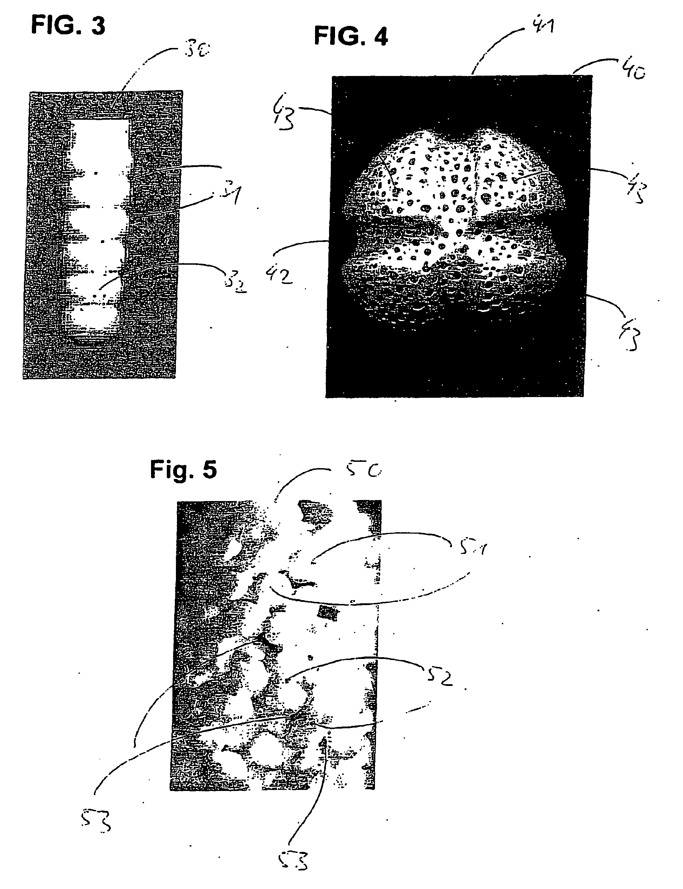

In Figur 3 ist ein erfindungsgemäßes Implantat dargestellt als Knochendübel 30, beispielsweise zur Refixation eines Bandes beim Kreuzbandersatz über die Aussenseite des Dübels, welches horizontale Einschnürungen 31 aufweist, die vorteilhaft jedoch auch schraubenförmig angeordnet sein können, und welches über die gesamte Oberfläche eine von Poren 32 unterbrochene äussere Schale aufweist.

In Figur 4 ist ein erfindungsgemäßes leichtes Keramikimplantat 40 dargestellt, net shaped gesintert, mit einer offenen Porosität über die gesamte Oberfläche 43, welches sich kreuzende Furchen oder Rinnen 41, 42 aufweist, in denen die Augenmuskeln vernäht werden können, welches als Teil eines künstlichen Auges verwendet wird.

Die Figur 5 zeigt ein Kugelkonglomerat 50, welches aus soliden Kugeln 51 besteht, welches erfindungsgemäß jedoch auch aus Elementen der Figuren 2a und 2b bestehen kann und sich durch breite Brücken 52 zwischen den Kugelelementen und regelmässige Zwischenkugelräume 53 auszeichnet.

## Patentansprüche

1. Gekammerter Implantatkörper (20), bestehend aus einer äusseren Schale mit glatter, rauher oder strukturierter Oberfläche (21), die über die Gesamt- oder Teilfläche durchbrochen ist von im Durchmesser bei der Herstellung einstellbaren runden oder rundlichen Poren, und einer inneren Struktur, bestehend aus einem Konglomerat aus von Kugelschalen (22) umgebenen Hohlräumen, wobei die Kugelschalen ein Schalengerüst bilden, das in direktem Zusammenhang mit der äusseren Schale steht, und wobei die Hohlräume ein zusammenhängendes Hohlraumsystem aus hohlen Kugeln oder kugelförmigen Hohlräumen bilden, dessen Hohlräume im Durchmesser bei der Herstellung einstellbar und durchgehend interkonnektierend verbunden sind, wobei das Hohlraumsystem der inneren Struktur durch die äussere Schale nach außen geöffnet ist,
**dadurch gekennzeichnet, dass** der Implantatkörper hergestellt ist mit einem Verfahren mit folgenden Verfahrensschritten:
Herstellen der äusseren Form des Implantatkörpers unter Berücksichtigung des Schrumpfungsfaktors des Werkstoffes des Schalengerüstes durch Füllen eines Werkzeugs mit unter Unterdruck expandierbaren formgebenden Elementen, wobei das Werkzeug eine oder mehrere Öffnungen zum Belüften aufweist,
Befüllen des Werkzeugs mit einer das Schalengerüst und die äussere Schale des Implantatkörpers bildenden Masse,
Beaufschlagen des Werkzeugs mit den formgebenden Elementen in einer geschlossenen Kammer mit einem Unterdruck, wobei das Beaufschlagen vor oder nach dem Befüllen des Werkzeugs mit der Masse erfolgt,
Entfernen der formgebenden Elemente,
Trocknen, Dehydratisieren, und Brennen des Implantatkörpers.

2. Gekammerter Implantatkörper (20), bestehend aus einer äusseren Schale mit glatter, rauher oder strukturierter Oberfläche (21), die über die Gesamt- oder Teilfläche durchbrochen ist von im Durchmesser bei der herstellung einstellbaren runden oder rundlichen Poren, und einer inneren Struktur, bestehend aus einem Konglomerat aus von Kugelschalen (22) umgebenen Hohlräumen, wobei die Kugelschalen ein Schalengerüst bilden, das in direktem Zusammenhang mit der äusseren Schale steht, und wobei die Hohlräume ein zusammenhängendes Hohlraumsystem aus hohlen Kugeln oder kugelförmigen Hohlräumen bilden, dessen Hohlräume im Durchmesser bei der herstellung einstellbar und durchgehend interkonnektierend verbunden sind, wobei das Hohlraumsystem der inneren Struktur durch die äussere Schale nach außen geöffnet ist,
**dadurch gekennzeichnet, dass** mindestens eine Pore der äusseren Schale in ihrem Durchmesser kleiner ist als der Durchmesser mindestens eines mit ihr in Verbindung stehenden Hohlraums des Hohlraumsystems, wobei das Verhältnis der Durchmesser 1:5 bis 1:1,5, im Mittel 1:2 bis 1:3 beträgt.

3. Implantatkörper nach Anspruch 1 oder 2 als Quader oder Würfel, Zylinder, Konus (30) oder Scheibe, Halbschale oder Halbkugel, Kugelsegment, Kugelschnitt, Keil oder Ring oder Scheibenring, Pyramide oder Pyramidenstumpf, Spirale oder Schraube.

4. Implantatkörper nach einem der Anspruche 1 bis 3, wobei die äussere Schale strukturiert ist in Form von zirkulären (31) oder radiären, longitudinalen, schrägen oder spiralförmigen, parallel oder sich kreuzend (41, 42) verlaufenden Einschnürungen, Furchen und/oder Wülsten, einzeln oder in Vielzahl, oder in Form einzelner oder mehrerer Erhebungen, Eindellungen, Zapfen oder anderer Strukturen.

5. Implantatkörper nach einem der Ansprüche 1-4, so beschaffen, dass die innere Struktur einem schalenförmigen Schwamm- oder Spongiosaknochen entspricht.

6. Implantatkörper nach Anspruch 1 in Form gekammerter Kugeln oder kugelförmiger Gebilde (20) einer Größe zwischen 0,8 mm und 12 mm im Durchmesser, im Mittel zwischen 1-2 mm und 3-6 mm im Durchmesser, und einem interkonnektierend verbundenen inneren Hohlraumsystem mit Durchmessern der Hohlräume (23) zwischen 80 µm und 1500 µm, vorzugsweise 150 µm bis 600 µm.

7. Implantatkörper nach einem der Ansprüche 1-6, wobei der Implantatkörper aus Einzelimplantaten ausgebildet ist, die mit einstellbar breiten Brücken (52) miteinander verbunden sind und ein in sich zusammenhängendes Hohlraumsystem (53) umschliessen, das es erlaubt, dass die vorzugsweise kugelförmigen Einzelimplantate in der Weise von Knochen umwachsen werden können, dass ein physiologisches Schwammknochengerüst entsteht, dessen Durchtritte durch die Verbindungsbrücken der Einzelimplantate vorgegeben sind.

8. Implantatkörper nach einem der Ansprüche 1-7, wobei der Gerüstwerkstoff ein Ca-Phosphat, insbesondere Tri-calciumphosphat oder Tetra-calciumphosphat, ein Hydroxylapatit, eine Calciumcarbonat- oder Calciumsulfat-Verbindung, oder eine Aluminiumoxyd-Keramik, ein Kollagen, eine Polyaminosäure oder ein anderes resorbierbares Polymer, ein Acrylat oder Abkömmling davon, ein Polymethylmethacrylat oder ein Copolymer davon, mit oder ohne Calciumphosphat, Calciumsulfat oder Calciumcarbonat als Füllwerkstoff, oder ein Metall, vorzugsweise eine CoCrMo-Legierung, Titan oder Tantal oder eine Legierung derselben ist.

9. Implantatkörper nach einem der Ansprüche 1-8, wobei das Hohlraumsystem ganz oder teilweise mit einem resorbierbaren oder nicht resorbierbaren organischen oder anorganischen Füllwerkstoff mit dämpfenden oder versteifenden Eigenschaften aufgefüllt oder beschichtet ist, und zwar in der Weise, dass die innere Füllung sich auf das Innere des Implantatkörpers beschränkt oder diesen vollständig ausfüllt, jedoch die äußere Schale nicht überragt, oder regelmäßig oder unregelmäßig aus den Poren der äußeren Schale hervorquillt und tropfenförmige und/oder kugelförmige oder ähnlich konfigurierte Erhebungen über die äußere Schale bildet, die gleichmäßig oder ungleichmäßig über die Oberfläche verteilt sind und Mikrobewegungen mehrerer gleicher oder ähnlicher Implantatkörper, beispielsweise in einer Schüttung, ganz oder nahezu ganz verhindern.

10. Implantatkörper nach Anspruch 9, wobei als Füllwerkstoff ein resorbierbares Polymer, beispielsweise eine Polyaminosäure, ein Polylactid, Polyglactin oder Glycosid, Polyacetale, Polysäureamide oder Polyester, oder ein nicht resorbierbares Polymer, wie Polymethylmethacrylate oder Agarose oder ein Gemisch aus Agarose und einem Calciumphosphat oder Calciumcarbonat verwandt wird.

11. Implantatkörper nach den Ansprüchen 1-10, wobei ausschließlich das Zentrum oder die Hohlräume entlang einer zentralen Achse des Implantatkörpers zusammenhängend oder nur einzelne Hohlräume oder Gruppen von Hohlräumen mit einem Füllwerkstoff nach den Ansprüchen 9 oder 10 aufgefüllt sind, der einen Wirkstoff enthält, der schnell oder langsam, kurzzeitig oder protrahiert freigesetzt wird und sich per Diffusion über die freie Oberfläche des Implantatkörpers zentrifugal verteilt.

12. Implantatkörper nach Anspruch 11, wobei der Wirkstoff ein Wachstumsfaktor, ein Gewebshormon und/oder ein Gemisch dieser Faktoren, beispielsweise als demineralisierte Knochenmatrix, oder ein Antibiotikum enthält oder ein Hormon, ein Immunsuppressivum, ein Zytostatikum oder ein Gemisch aus Wachstumsfaktoren und einem Antibiotikum oder eine Kombination aus verschiedenen Wirkstoffen, welche aus dem Träger-Füllwerkstoff freigesetzt werden.

13. Implantatkörper nach Anspruch 12, wobei als Antibiotikum Gentamycin, Clindamycim Streptomycin oder ein anderes bakteriostatisch oder bakterizid wirkendes Mittel verwandt wird.

14. Implantatkörper nach einem der Ansprüche 1-13, wobei in standardisierter Weise weite und engere Hohlraumsysteme ausgebildet sind, die zusammenhängend oder unterbrochen, einzeln oder in Gruppen oder auch entlang einer Achse angeordnet sind und unterschiedlich weite Interkonnexionen aufweisen, und die Hohlräume sich damit leichter oder schwerer mit Füllwerkstoffen oder Wirkstoffen oder beidem ausfällen lassen.

15. Implantatkörper nach den Ansprüchen 1-14, wobei verschieden große Hohlräume mit einem interkonnektierenden Gerüst aus einem anderen resorbierbaren oder nicht
resorbierbaren Werkstoff aufgebaut sind, beispielsweise ein großer Hohlraum mit einem schalenförmigen Gerüst aus Hydroxylapatit mit einem schalenförmig aufgebauten Gerüst aus einem leicht resorbierbaren Tricalciumphosphat, sodass in dieser Weise ein durchgehend kombinierter, beispielsweise aus Hydroxylapatit und Tricalciumphosphat bestehender oder ein aus anderen Werkstoffen kombinierter Implantatkörper entsteht.

16. Verbundimplantatkörper, bestehend aus einem Implantatkörper I nach einem der Ansprüche 1-15, und einem Implantatkörper II nach einem dieser Ansprüche, jedoch mit sich unterscheidender Porosität, wobei die Implantatkörper I und II in der Weise verbunden sind, dass letzterer entlang einer zentralen Achse des ersteren mechanisch eingesetzt ist, wobei vorzugsweise zuvor aus dem Implantatkörper I durch Schleifen oder eine andere Trennmethode ein zentraler Zylinder herausgenommen wurde oder dieser durch das Herstellverfahren des Implantatkörpers I bereits ausgespart ist.

17. Verbundimplantatkörper nach Anspruch 16, wobei der in den Implantatkörper I mechanisch eingesetzte Implantatkörper II aus einem porösen oder soliden Werkstoff mit oder ohne Wirkstoff besteht und entweder resorbierbar oder nicht resorbierbar ist.

18. Verfahren zur Herstellung eines Implantatkörpers nach Anspruch 2 mit folgenden Verfahrensschritten:
Herstellen der äusseren Form des Implantatkörpers unter Berücksichtigung des Schrumpfungsfaktors des Werkstoffes des Schalengerüstes durch Füllen eines Werkzeugs mit unter Unterdruck expandierbaren formgebenden Elementen, wobei das Werkzeug eine oder mehrere Öffnungen zum Belüften aufweist,
Befüllen des Werkzeugs mit der das Schalengerüst und die äussere Schale des Implantatkörpers bildenden Masse,
Beaufschlagen des Werkzeugs mit den formgebenden Elementen in einer geschlossenen Kammer mit einem Unterdruck, wobei das Beaufschlagen vor oder nach dem Befüllen des Werkzeugs mit der Masse erfolgt,
Entfernen der formgebenden Elemente,
Trocknen, Dehydratisieren, und Brennen des Implantatkörpers.

19. Verfahren nach Anspruch 18, wobei als Werkzeug eine deformierbare Form verwendet wird.

20. Verfahren nach Anspruch 19, wobei für die deformierbare Form ein plastisch oder elastisch deformierbarer Werkstoff verwendet wird.

## Claims

1. A chambered implant body (20) comprising an outer shell having a smooth, rough or structured surface (21) which is perforated over the entire or partial area by round or roundish pores whose diameter is adjustable during manufacturing, and an inner structure formed from a conglomerate of cavities surrounded by ball shells (22), wherein the ball shells form a shell frame having a direct connection to the outer shell, and wherein the cavities form a continuous cavity system formed from hollow balls or ball-shaped cavities, the diameter of the cavities of said cavity system being adjustable during manufacturing and being continuously interconnected, wherein the cavity system of the inner structure is opened through the outer shell to the outside,
**characterized in that** the implant body is manufactured by means of a method comprising the following method steps:
preparing the outer shape of the implant body considering the shrinking factor of the material of the shell frame by filling a mold with shaping elements being expandable under underpressure, wherein the mold comprises one or multiple openings for venting;
filling the mold with a material forming the shell frame and the outer shell of the implant body;
applying underpressure to the mold with the shaping elements in a closed chamber,
wherein the applying is performed prior to or after the filling of the mold with the material;
removing the shaping elements;
drying, dehydrating and calcinating the implant body.

2. A chambered implant body (20) comprising an outer shell having a smooth, rough or structured surface (21) which is perforated over the entire or partial area by round or roundish pores whose diameter is adjustable during manufacturing, and an inner structure formed from a conglomerate of cavities surrounded by ball shells (22), wherein the ball shells form a shell frame having a direct connection to the outer shell, and wherein the cavities form a continuous cavity system formed by hollow balls or ball-shaped cavities, the diameter of the cavities of said cavity system being adjustable during manufacturing and being continuously interconnected, wherein the cavity system of the inner structure is opened through the outer shell to the outside,
**characterized in that** at least one pore of the outer shell has a smaller diameter than the diameter of at least one cavity of the cavity system connected therewith, wherein the ratio of the diameters is 1:5 to 1:1.5, on average 1:2 to 1:3.

3. The implant body according to claim 1 or 2, shaped as a cuboid or cube, cylinder, cone (30) or disc, half shell or halfball, ball segment, ball section, wedge or ring or disc ring, pyramid or fustrum of pyramid, spiral or screw.

4. The implant body according to any one of claims 1 to 3, wherein the outer shell is structured in the shape of a circular (31) or radial, longitudinal, oblique or helical, parallel or crossing (41, 42) extending contractions, channels and/or bulges, singular or in a plurality thereof, or in the shape of single or plural elevations, recesses, pins or other structures.

5. The implant body of any one of claims 1 to 4 structured such that the inner structure corresponds to a bowl-shaped sponge bone or cancellous bone.

6. The implant body according to claim 1 in the form of chambered balls or ball-shaped structures (20) of a size between 0.8 mm and 12 mm in diameter, on average between 1-2 mm and 3-6 mm in diameter, and an interconnectingly connected inner cavity system having diameters of the cavities (23) between 80 µm and 1,500 µm, preferably 150 µm to 600 µm.

7. The implant body according to any one of claims 1 to 6, wherein the implant body is formed from individual implants which are connected to each other via bridges (52) having adjustable widths and encompass a cavity system (53) being interrelated, which enables bones to grow around the preferably ball-shaped individual implant bodies in a manner that a physiological sponge bone frame is generated, the passages of which are determined by the connecting bridges of the individual implant bodies.

8. The implant body according to any one of claims 1 to 7, wherein the frame material is a calciumphosphate, especially tri-calciumphosphate or tetra-calciumphosphat, hydroxyapatite, a calciumcarbonate or calcium-sulfate-composition, or an aluminium oxide ceramic, a collagen, a polyaminoacid or any other resorbable polymer, an acrylate or derivate thereof, a polymethyl-methacrylate or a copolymer thereof, with or without calciumphosphate, calcium sulphate or calcium carbonate as a filler, or a metal, preferably a CoCrMo alloy, titanium or tantalum or an alloy thereof.

9. The implant body according to any one of claims 1 to 8, wherein the cavity system is filled or coated completely or partially with an resorbable or non-resorbable organic or inorganic filler material having dampening or reinforcing properties, in a manner that the inner filling is restricted to the inside implant body or completely fills the same, but does not extend beyond the outer shell, or regularly or irregularly wells out of the pores of the outer shell to form drop-shaped and/or ball-shaped or similarly shaped elevations over the outer shell which are regularly or irregularly distributed over the surface and completely or nearly completely prevent micro-movements of plural identical or similar implant bodies, for example in a fill.

10. The implant body according to claim 9, wherein a resorbable polymer, e.g. a polyaminoacid, a polyactide, polyglactine or glycoside, polyacetales, polyacidamides or polyester, or a non-resorbable polymer like polymethyl-methacrylates or agarose or a mixture of agarose and a calciumphosphate or calciumcarbonate is used.

11. The implant body according to any one of claims 1 to 10, wherein exclusively the center or the cavities along the center axis of the implant body, interrelated or individually, or groups of cavities, are filled with a filler material according to claims 9 or 10, said filler material comprising an active agent which is released fast or slowly, for a short time or in a retarded way and centrifugally distributes by diffusion over the free surface of the implant body.

12. The implant body according to claim 11, wherein the active agent comprises a growth factor, a tissue hormone and/or a mixture thereof, e.g. as a demineralised bone matrix, or an antibiotic or a hormone, an immunosuppressive drug, a cytostatic or a mixture of growth factors and an antibiotic or a combination of different active agents which are released from the carrier filling material.

13. The implant body according to claim 12, wherein gentamycin, clindamycin, streptomycin or any other bacteriostatic or bactericide agent is used as the antibiotic.

14. The implant body according to any one of claims 1 to 13, wherein wide and narrow cavity systems are formed therein in a standardized manner, that are arranged in an interrelated or interrupted manner, individually or in groups or also along an axis and comprise interconnections of different widths and, thus, the cavities can be filled more or less easily with filler materials or active agents or both.

15. The implant body according to any one of claims 1 to 14, wherein cavities of different sizes are structured with an interconnecting frame of another resorbable or non-resorbable material, e.g., a large cavity having a shell-shaped frame made of hydroxyapatite having a shell-shaped structured frame of an easily resorbable tri-calciumphosphate, so that thus a continuously combined implant body consisting of, e.g., hydroxyapatite and tri-calciumphosphate, or an implant body combined of other materials is generated.

16. A composite implant body comprising an implant body I according to any one of claims 1 to 15 and an implant body II according to any one of these claims but having differing porosities, wherein the implant bodies I and II are connected in such a manner that the latter is mechanically inserted along a central axis of the former, wherein, preferably in advance, a central cylinder has been removed from implant body I by grinding or another cutting process, or said cylinder has already been left open when manufacturing implant body I.

17. The composite implant body according to claim 16, wherein the implant body II inserted mechanically into implant body I is made from a porous or solid material comprising or not an active agent and being either resorbable or non-resorbable.

18. A method for manufacturing an implant body according to claim 2 comprising the following steps:
preparing the outer shape of the implant body considering the shrinking factor of the material of the shell frame by filling a mold with shaping elements being expandable under underpressure, wherein the mold comprises one or multiple openings for venting;
filling the mold with a material forming the shell frame and the outer shell of the implant body;
applying underpressure to the mold with the shaping elements in a closed chamber,
wherein the applying is performed prior to or after the filling of the mold with the material;
removing the shaping elements;
drying, dehydrating and calcinating the implant body.

19. The method according to claim 18, wherein a deformable mold is used as the mold.

20. The method according to claim 19, wherein a plastically or elastically deformable material is used for the deformable mold.

## Revendications

1. Corps d'implant (20) compartimenté, constitué d'une coque externe à surface (21) lisse, rugueuse ou structurée, perforée sur toute sa surface ou une partie de sa surface par des pores circulaires ou arrondis ajustables en diamètre lors de la fabrication, et d'une structure interne, formée d'un conglomérat de cavités entourées de coques sphériques (22), lesdites coques sphériques formant une ossature de coques en liaison directe avec la coque externe, et les cavités formant un système cohérent de cavités constitué de sphères creuses ou de cavités sphériques, lesdites cavités étant ajustables en diamètre lors de la fabrication et interconnectées entre elles de manière continue, le système de cavités de la structure interne étant ouvert vers l'extérieur par la coque externe,
**caractérisé en ce que** ledit corps d'implant est fabriqué au moyen d'un procédé comportant les étapes de procédé suivantes :
fabrication de la forme externe du corps d'implant, en tenant compte du facteur de contraction du matériau de l'ossature de coques, en remplissant un moule avec des éléments de formage expansibles par dépression, le moule comportant une ou plusieurs ouvertures d'aération,
remplissage de le moule avec une masse formant l'ossature de coques et la coque externe du corps d'implant,
exposition de le moule avec les éléments de formage à une dépression dans un compartiment clos, l'exposition ayant lieu avant ou après le remplissage de le moule avec la masse,
retrait des éléments de formage,
séchage, déshydratation et cuisson du corps d'implant.

2. Corps d'implant (20) compartimenté, constitué d'une coque externe à surface (21) lisse, rugueuse ou structurée, perforée sur toute sa surface ou une partie de sa surface par des pores circulaires ou arrondis ajustables en diamètre lors de la fabrication, et d'une structure interne, formée d'un conglomérat de cavités entourées de coques sphériques (22), lesdites coques sphériques formant une ossature de coques en liaison directe avec la coque externe, et les cavités formant un système cohérent de cavités constitué de sphères creuses ou de cavités sphériques, lesdites cavités étant ajustables en diamètre lors de la fabrication et interconnectées entre elles de manière continue, le système de cavités de la structure interne étant ouvert vers l'extérieur par la coque externe,
**caractérisé en ce qu'**au moins un pore de la coque externe présente un diamètre inférieur au diamètre d'au moins une cavité des système de cavités qui lui est reliée, le rapport des diamètres étant compris entre 1:5 et 1:1,5, en moyenne entre 1:2 et 1:3.

3. Corps d'implant selon la revendication 1 ou la revendication 2, réalisé comme parallélépipède ou cube, cylindre, cône (30) ou disque, demi-coque ou hémisphère, calotte sphérique, segment sphérique, coin, anneau ou tore, pyramide ou tronc de pyramide, spirale ou hélice.

4. Corps d'implant selon l'une des revendications 1 à 3, où la coque externe est structurée sous la forme d'étranglements, de gorges et/ou de boudins circulaires (31) ou radiaux, longitudinaux, obliques ou hélicoïdaux, parallèles entre eux ou se croisant (41, 42), isolés ou en nombre, ou sous la forme de reliefs, d'empreintes, d'axes ou d'autres structures, isolés ou en nombre.

5. Corps d'implant selon l'une des revendications 1 à 4, de telle nature que la structure interne correspond à un os spongieux en forme de coque.

6. Corps d'implant selon la revendication 1 sous la forme de sphères compartimentées ou d'entité sphérique (20) de diamètre compris entre 0,8 mm et 12 mm, en moyenne entre 1-2 mm et 3-6 mm, et d'un système interne de cavités interconnectées, avec des diamètres de cavités (23) compris entre 80 µm et 1500 µm, de préférence entre 150 µm et 600 µm.

7. Corps d'implant selon l'une des revendications 1 à 6, où le corps d'implant est constitué d'implants individuels, reliés entre eux par des ponts (52) de largeur ajustable et qui entourent un système cohérent de cavités (53), lequel permet une croissance périphérique des implants individuels préférentiellement sphériques à la manière d'os, pour former une ossature spongieuse, dont les passages sont définis par les ponts de liaison des implants individuels.

8. Corps d'implant selon l'une des revendications 1 à 7, où le matériau d'ossature est un phosphate de calcium, en particulier tricalcium phosphate ou tétracalcium phosphate, une hydroxylapatite, un composé de carbonate de calcium ou de sulfate de calcium, ou une céramique d'oxyde d'aluminium, un collagène, un acide polyaminé ou un autre polymère résorbable, un acrylate ou un dérivé d'acrylate, un polyméthacrylate de méthyle ou un copolymère de celui-ci, avec ou sans phosphate de calcium, sulfate de calcium ou carbonate de calcium comme matière de charge, ou un métal, de préférence un alliage CoCrMo, le titane ou le tantale ou un alliage de ceux-ci.

9. Corps d'implant selon l'une des revendications 1 à 8, où le système de cavités est totalement ou partiellement rempli ou revêtu de matière de charge organique ou inorganique, résorbable ou non résorbable, ayant des propriétés d'amortissement ou de renforcement, et cela de telle manière que la charge interne reste limitée à l'intérieur du corps d'implant ou remplisse totalement celui-ci mais sans dépasser de la coque externe, ou sorte régulièrement ou irrégulièrement des pores de la coque externe et forme des reliefs en forme de gouttes et/ou sphériques ou de configuration analogue sur la coque externe, répartis de manière homogène ou inhomogène à la surface et inhibant totalement ou presque totalement des micro-déplacements de plusieurs corps d'implant identiques ou similaires, par exemple dans une charge.

10. Corps d'implant selon la revendication 9, où la matière de charge utilisée est un polymère résorbable, tel qu'un acide polyaminé, un polylactide, polyglactine ou glycoside, polyacétale, polyacide amique ou polyester, ou un polymère non résorbable, tel que polyméthacrylate de méthyle ou agarose ou un mélange d'agarose et d'un phosphate de calcium ou carbonate de calcium.

11. Corps d'implant selon les revendications 1 à 10, où exclusivement le centre ou les cavités connexes le long d'un axe central du corps d'implant, ou exclusivement des cavités ou groupes de cavités sont remplis de matière de charge selon la revendication 9 ou la revendication 10, laquelle contient une substance active libérée rapidement ou lentement, à court terme ou à libération différée, et se répartit de manière centrifuge par diffusion sur la surface libre du corps d'implant.

12. Corps d'implant selon la revendication 11, où la substance active contient un facteur de croissance, une hormone tissulaire et/ou un mélange de ces facteurs, par exemple sous forme de matrice osseuse déminéralisée, ou un antibiotique ou une hormone, un immunosuppresseur, un cytostatique ou un mélange de facteurs de croissance et d'un antibiotique ou une combinaison de différentes substances actives, libérées par la matière de charge support.

13. Corps d'implant selon la revendication 12, où l'antibiotique utilisé est la gentamycine, la clindamycine, la streptomycine ou un autre agent à effet bactériostatique ou bactéricide.

14. Corps d'implant selon l'une des revendications 1 à 13, où des systèmes de cavités larges et étroits sont réalisés de manière standardisée, lesquels sont disposés de manière cohérente ou interrompue, isolément ou en groupes ou aussi le long d'un axe, et présentent des interconnexions de grandeurs différenciées, et où les cavités se laissent ainsi combler plus facilement ou plus difficilement par des matières de charge ou des substances actives, ou par les deux.

15. Corps d'implant selon les revendications 1 à 14, où des cavités de grandeurs différentes sont produites avec une ossature interconnectée d'un autre matériau résorbable ou non résorbable, par exemple une grande cavité avec une ossature d'hydroxylapatite en forme de coque avec une ossature en forme de coque de tricalcium phosphate facilement résorbable, de manière à former ainsi un corps d'implant combiné de manière continue, constitué notamment d'hydroxylapatite et de tricalcium phosphate ou un corps d'implant combiné d'autres substances.

16. Corps d'implant composite, composé d'un corps d'implant I selon l'une des revendications 1 à 15, et d'un corps d'implant II selon l'une desdites revendications, pourvu toutefois d'une porosité différenciée, où lesdits corps d'implant I et II sont raccordés de telle manière que le corps d'implant II est mécaniquement mis en place le long d'un axe central du corps d'implant I, un cylindre central ayant de préférence été préalablement retiré du corps d'implant 1 par meulage ou par un autre procédé de séparation, ou celui-ci ayant déjà été ménagé par le procédé de fabrication du corps d'implant I.

17. Corps d'implant composite selon la revendication 16, où le corps d'implant II mis en place mécaniquement dans le corps d'implant I est constitué d'un matériau poreux ou solide avec ou sans substance active, et est résorbable ou non résorbable.

18. Procédé de fabrication d'un corps d'implant selon la revendication 2, comportant les étapes de procédé suivantes :
fabrication de la forme externe du corps d'implant, en tenant compte du facteur de contraction du matériau de l'ossature de coques, en remplissant un moule avec des éléments de formage expansibles par dépression, le moule comportant une ou plusieurs ouvertures d'aération,
remplissage de le moule avec une masse formant l'ossature de coques et la coque externe du corps d'implant,
exposition de le moule avec les éléments de formage à une dépression dans un compartiment clos, l'exposition ayant lieu avant ou après le remplissage de le moule avec la masse,
retrait des éléments de formage,
séchage, déshydratation et cuisson du corps d'implant.

19. Procédé selon la revendication 18, où un moule déformable est employé comme outil.

20. Procédé selon la revendication 19, où un matériau plastique ou élastiquement déformable est utilisé pour le moule déformable.
